# EUROPEAN PATENT APPLICATION

(11) **EP 4 473 966 A1**
(43) Date of publication of application: **11.12.2024**
(21) Application number: 23749766.4
(22) Date of filing: 01.02.2023
(51) Int. Cl.: A61K 31/444, A61K 31/5025, A61K 45/00, A61P 35/00, A61P 35/02, A61P 43/00

(54) **CANCER TREATMENT AGENT INCLUDING MALT1 INHIBITING DRUG AS ACTIVE INGREDIENT**

(30) Priority: 02.02.2022 JP 2022015169
(71) Applicant: ONO Pharmaceutical Co., Ltd., Osaka-shi, Osaka 541-8526 (JP); Chordia Therapeutics Inc., Fujisawa-shi, Kanagawa 251-0012 (JP)
(72) Inventor: KOZAKI, Ryohei, Mishima-gun, Osaka 618-8585 (JP); FUJIKAWA, Ryu, Mishima-gun, Osaka 618-8585 (JP); ONO, Kazuya, Mishima-gun, Osaka 618-8585 (JP); MORISHITA, Daisuke, Fujisawa-shi, Kanagawa 251-0012 (JP); SATOH, Yoshihiko, Fujisawa-shi, Kanagawa 251-0012 (JP); MIZUTANI, Akio, Fujisawa-shi, Kanagawa 251-0012 (JP)
(74) Representative: Hoffmann Eitle
(86) International application number: PCT/JP2023/003154
(87) International publication number: WO 2023/149450

(57) **Abstract**

A combination of a MALT1 inhibitor which is a compound represented by formula (I) described in the description, a salt thereof, or a co-crystal, a hydrate or a solvate thereof, and a cytotoxic anticancer agent and/or a molecular targeted drug exhibits a strong antitumor effect, and thus is useful for treating hematological cancer.

## Description

### TECHNICAL FIELD

The present disclosure relates to a pharmaceutical composition containing a MALT1 inhibitor as an active ingredient for treating hematological cancer.

### BACKGROUND ART

In the major T cells and B cells responsible for cell-mediated immunity, the T-cell receptor signal and B-cell receptor signal play important roles in their function. Transduction abnormality of these signals causes various diseases such as cancer and inflammatory disease. In fact, in the case of cancer, it has been reported that genetic analysis of patients with T cell-derived lymphoma such as ATL (adult T cell leukemia lymphoma), which is one of refractory lymphomas, reveals a genetic abnormality in the T-cell receptor signal/NT-κB pathway, and that the B-cell receptor signaling pathway/NF-κB pathway is also persistently activated in other B-cell lymphomas such as DLBCL (diffuse large B-cell lymphoma) and MCL (mantle cell lymphoma).

A CBM protein complex where these T-cell and B-cell receptor signals join together is composed of scaffold protein CARD11, adaptor protein BCL10, and MALT1(Mucosa associated lymphoid tissue lympoma translocation protein 1) having paracaspase activity. The formation of the CBM protein complex is promoted by the T-cell receptor signal and the B-cell receptor signal, leading to an enhancement of the paracaspase activity of MALT1 and activating the transcription factor NF-κB.

Accordingly, an inhibitor that inhibits the activity of MALT1 is expected to be able to correct the enhancement of the activity of MALT 1 caused by abnormalities in T-cell receptor signal and B-cell receptor signal, and is considered to be useful as a prophylactic or therapeutic agent against cancer, inflammatory disease and the like caused by the activity of MALT1 (see Non Patent Literatures 1 to 3).
Patent Literature 1 discloses that a compound represented by a formula (I) described later has a MALT1 inhibitory action and is useful as a therapeutic agent against cancer (see Patent Literature 1).

### CITATIONS LIST

### Patent Literature

Patent Literature 1: WO 2020/111087 A

### Non Patent Literatures

Non Patent Literature 1: Clinical Cancer Research, Vol. 19, No. 24, p. 6662-6668, 2013
Non Patent Literature 2: Cellular and Molecular Life Sciences, Vol. 73, p. 459-473, 2016
Non Patent Literature 3: Current Opinion in Hematology, Vol. 23, No. 4, p. 402-409, 2016

### SUMMARY OF INVENTION

### TECHNICAL PROBLEMS

One of the objects of the present disclosure is to find a combination of drugs useful for hematological cancer treatment and provide the combination as a drug.

### SOLUTIONS TO PROBLEMS

In order to solve the above problems, the present inventors have found that the above problems can be solved by a combination of a MALT1 inhibitor, which is a compound represented by the following formula (I), a salt thereof, or a co-crystal, a hydrate or a solvate thereof, and an anticancer agent (hereinafter, it may be abbreviated as a combination of the present invention.).

Therefore, in one embodiment, there is provided a pharmaceutical composition containing, as an active ingredient, a MALT1 inhibitor which is a compound represented by the following formula (I), a salt thereof, or a co-crystal, a hydrate or a solvate thereof, used in combination with an anticancer agent, for treating a hematological cancer patient.

### ADVANTAGEOUS EFFECTS OF INVENTION

The combination of the present invention is useful for hematological cancer treatment.

### DESCRIPTION OF EMBODIMENTS

Unless specifically defined otherwise, the terms used herein have the meanings as commonly understood by one of ordinary skill in the art of organic chemistry, medicine, pharmacy, molecular biology, microbiology, and the like. Definitions of several terms used herein are described below. The definitions herein take precedence over the general understanding.

As used herein, a numerical value accompanied with the term "about" is intended to include any value within the range of ±10% of the value. For example, "about 10" shall include "9 to 11". A range of numerical values includes all numerical values between both endpoints and numerical values of the both endpoints. The term "about" used for a range applies to both endpoints of the range. Therefore, for example, "about 10 to 20" shall include "9 to 22".

### (1) MALT1 inhibitor

In one embodiment, the MALT1 inhibitor used in the combination of the present invention is a compound represented by a formula (I) described in Patent Literature 1 (WO 2020/111087 A): 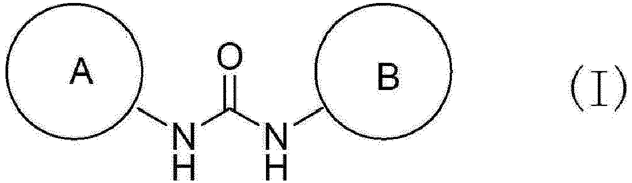 (wherein
A represents 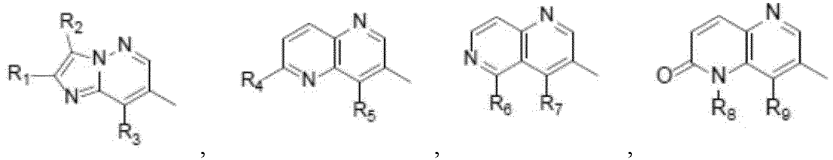 , or 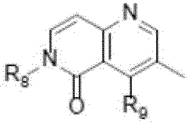
R₁ represents 1) a hydrogen atom, 2) a halogen atom, 3) a cyano group, 4) a C₁₋₃ alkyl group which may be substituted with 1 to 3 halogen atoms, 5) a C₁₋₃ alkoxy group, 6) a C₃₋₆ cycloalkyl group, or 7) a phenyl group;
R₂ represents 1) a hydrogen atom or 2) a halogen atom;
R₃ represents 1) a C₁₋₆ alkyl group which may be substituted with 1 to 3 substituents selected from a C₁₋₃ alkoxy group, a hydroxyl group and a halogen atom, 2) a pyrazolyl group which may be substituted with 1 to 3 substituents selected from a C₁₋₃ alkyl group and a halogen atom, 3) a C₃₋₆ cycloalkyl group, 4) an amino group di-substituted with a C₁₋₃ alkyl group, or 5) a phenyl group which may be substituted with 1 to 3 halogen atoms;
R₄ and R₆ represent 1) a hydrogen atom, 2) a halogen atom, 3) a C₁₋₃ alkyl group which may be substituted with 1 to 3 substituents selected from a) a hydroxyl group, b) a C₁₋₃ alkoxy group which may be substituted with a 4-methoxyphenyl group and c) a halogen atom, or 4) a C₁₋₃ alkoxy group which may be substituted with 1 to 3 halogen atoms;
R₅, R₇ and R₉ represent 1) a C₁₋₆ alkyl group which may be substituted with 1 to 3 C₁₋₃ alkoxy groups or 2) a phenyl group which may be substituted with 1 to 3 halogen atoms;
R₈ represents a C₁₋₃ alkyl group; and
B represents
   1) a phenyl group which may be substituted with 1 to 3 substituents selected from a) a halogen atom, b) a cyano group, c) a C₁₋₃ alkoxy group which may be substituted with 1 to 3 halogen atoms, and d) a triazolyl group,
   2) a C₃₋₆ cycloalkyl group which may be substituted with 1 to 3 substituents selected from a) a C₁₋₃ alkyl group which may be substituted with 1 to 3 halogen atoms and b) a halogen atom,
   3) a pyridyl group which may be substituted with 1 to 3 substituents selected from a) a halogen atom, b) a cyano group, c) a C₁₋₃ alkyl group which may be substituted with 1 to 3 halogen atoms, d) a C₁₋₃ alkoxy group which may be substituted with 1 to 3 substituents selected from a halogen atom and a C₁₋₃ alkoxy group, e) a pyrazolyl group which may be substituted with 1 to 3 C₁₋₃ alkyl groups, f) an imidazolyl group which may be substituted with 1 to 3 C₁₋₃ alkyl groups, g) a triazolyl group which may be substituted with 1 to 3 C₁₋₃ alkyl groups which may be substituted with 1 to 3 substituents selected from a C₁₋₃ alkoxy group and a halogen atom, h) an azetidinyl group, i) a pyrrolidonyl group, j) a tetrazolyl group which may be substituted with 1 to 3 C₁₋₃ alkyl groups, k) a pyrimidinyl group, and l) an oxazolyl group,
   4) a pyrazolyl group which may be substituted with 1 to 3 substituents selected from a) a C₁₋₃ alkyl group which may be substituted with 1 to 3 halogen atoms, b) a C₁₋₃ alkoxy group which may be substituted with 1 to 3 halogen atoms, c) a cyano group, and d) a halogen atom, or
   5) an imidazopyridyl group which may be substituted with 1 to 3 halogen atoms)
or a salt thereof, or a co-crystal, a hydrate or a solvate thereof (sometimes abbreviated as "Compound (I)" in the description).

Another embodiment of the MALT1 inhibitor includes a compound described in WO2015/181747 A, WO2017/081641 A, WO2018/020474 A, WO2018/085247 A, WO2018/119036 A, WO2018/141749 A, WO2019/243965 A, WO2021/000855 A, or WO2021/134004 A.

In the description, examples of the "halogen atom" include fluorine, chlorine, bromine and iodine.

In the description, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

In the description, examples of the "C₃₋₆ cycloalkyl group" include cyclopropyl, cyclobutyl, cyclopentyl and cyclohexyl.

In the description, examples of the "C₁₋₃ alkoxy group" include methoxy, ethoxy, propoxy and isopropoxy.

In the description, examples of the "amino group di-substituted with a C₁₋₃ alkyl group" include dimethylamino, ethylmethylamino, diethylamino, ethylpropylamino and dipropylamino.

In the description, the "C₁₋₃ alkyl group" includes the above "C₁₋₆ alkyl group" having 1 to 3 carbon atoms.

Hereinafter, a detailed description is made of the definition of each symbol in the formula (I).
A represents 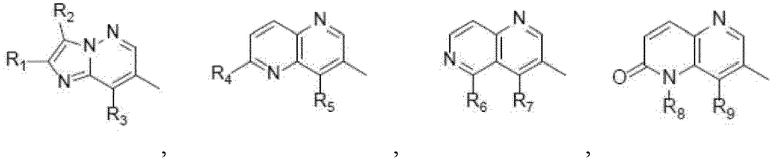 , or 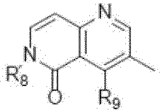
A is preferably

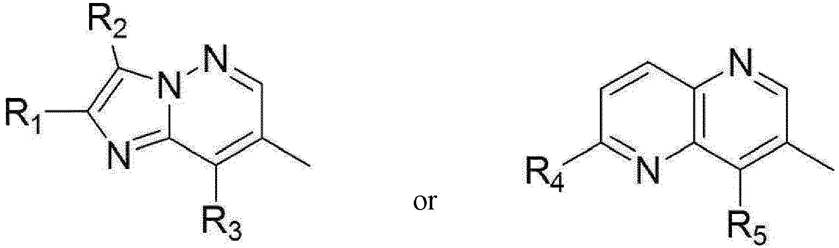

In one preferred embodiment of the present invention, A is 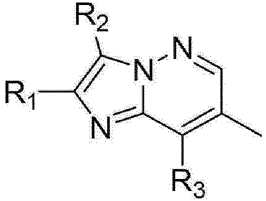 , and in another preferred embodiment of the present invention, A is 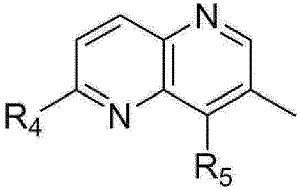
R₁ represents 1) a hydrogen atom, 2) a halogen atom (e.g., chlorine atom, bromine atom), 3) a cyano group, 4) a C₁₋₃ alkyl group (e.g., methyl) which may be substituted with 1 to 3 halogen atoms (e.g., fluorine atom), 5) a C₁₋₃ alkoxy group (e.g., methoxy), 6) a C₃₋₆ cycloalkyl group (e.g., cyclopropyl), or 7) a phenyl group.
R₁ is more preferably 1) a halogen atom (e.g., chlorine atom) or 2) a C₁₋₃ alkyl group (e.g., methyl).
R₂ represents 1) a hydrogen atom or 2) a halogen atom (e.g., fluorine atom, chlorine atom).
R₂ is preferably a hydrogen atom.
R₃ represents 1) a C₁₋₆ alkyl group (e.g., ethyl, isopropyl, sec-butyl) which may be substituted with 1 to 3 substituents selected from a C₁₋₃ alkoxy group (e.g., methoxy), a hydroxyl group and a halogen atom (e.g., fluorine atom), 2) a pyrazolyl group (e.g., 4-pyrazolyl) which may be substituted with 1 to 3 substituents selected from a C₁₋₃ alkyl group (e.g., methyl) and a halogen atom (e.g., chlorine atom), 3) a C₃₋₆ cycloalkyl group (e.g., cyclopropyl), 4) an amino group di-substituted with a C₁₋₃ alkyl group (e.g., methyl), or 5) a phenyl group which may be substituted with 1 to 3 halogen atoms (e.g., chlorine atom).
R₃ is preferably a C₁₋₆ alkyl group (e.g., ethyl, isopropyl) which may be substituted with 1 to 3 C₁₋₃ alkoxy groups (e.g., methoxy).
R₄ and R₆ represent 1) a hydrogen atom, 2) a halogen atom (e.g., chlorine atom), 3) a C₁₋₃ alkyl group (e.g., methyl, ethyl) which may be substituted with 1 to 3 substituents selected from a) a hydroxyl group, b) a C₁₋₃ alkoxy group (e.g., methoxy) which may be substituted with a 4-methoxyphenyl group and c) a halogen atom (e.g., fluorine atom), or 4) a C₁₋₃ alkoxy group (e.g., methoxy, ethoxy) which may be substituted with 1 to 3 halogen atoms (e.g., fluorine atom).
R₄ is preferably 1) a halogen atom (e.g., chlorine atom) or 2) a C₁₋₃ alkyl group (e.g., methyl).
R₆ is preferably 1) a hydrogen atom, 2) a halogen atom (e.g., chlorine atom), 3) a C₁₋₃ alkyl group (e.g., methyl) or 4) a C₁₋₃ alkoxy group (e.g., methoxy).
R₅, R₇ and R₉ represent 1) a C₁₋₆ alkyl group (e.g., ethyl, isopropyl) which may be substituted with 1 to 3 C₁₋₃ alkoxy groups (e.g., methoxy, ethoxy) or 2) a phenyl group which may be substituted with 1 to 3 halogen atoms (e.g., chlorine atom).
R₅ is preferably a C₁₋₆ alkyl group (e.g., ethyl) which may be substituted with 1 to 3 C₁₋₃ alkoxy groups (e.g., methoxy).
R₇ is preferably 1) a C₁₋₆ alkyl group (e.g., ethyl, isopropyl) which may be substituted with 1 to 3 C₁₋₃ alkoxy groups (e.g., methoxy) or 2) a phenyl group which may be substituted with 1 to 3 halogen atoms (e.g., chlorine atom).
R₉ is preferably a C₁₋₃ alkyl group (e.g., ethyl) which may be substituted with 1 to 3 C₁₋₃ alkoxy groups (e.g., methoxy).
R₈ represents a C₁₋₃ alkyl group (e.g., methyl).
B represents
   1) a phenyl group which may be substituted with 1 to 3 substituents selected from a) a halogen atom (e.g., fluorine atom, chlorine atom), b) a cyano group, c) a C₁₋₃ alkoxy group (e.g., methoxy) which may be substituted with 1 to 3 halogen atoms (e.g., fluorine atom), and d) a triazolyl group,
   2) a C₃₋₆ cycloalkyl group (e.g., cyclohexyl) which may be substituted with 1 to 3 substituents selected from a) a C₁₋₃ alkyl group (e.g., methyl) which may be substituted with 1 to 3 halogen atoms (e.g., fluorine atom) and b) a halogen atom (e.g., fluorine atom),
   3) a pyridyl group which may be substituted with 1 to 3 substituents selected from a) a halogen atom (e.g., fluorine atom, chlorine atom, bromine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy, ethoxy) which may be substituted with 1 to 3 substituents selected from a halogen atom (e.g., fluorine atom) and a C₁₋₃ alkoxy group (e.g., methoxy), e) a pyrazolyl group which may be substituted with 1 to 3 C₁₋₃ alkyl groups (e.g., methyl), f) an imidazolyl group which may be substituted with 1 to 3 C₁₋₃ alkyl groups (e.g., methyl), g) a triazolyl group which may be substituted with 1 to 3 C₁₋₃ alkyl groups (e.g., methyl) which may be substituted with 1 to 3 substituents selected from a C₁₋₃ alkoxy group (e.g., methoxy) and a halogen atom (e.g., fluorine atom), h) an azetidinyl group, i) a pyrrolidonyl group, j) a tetrazolyl group which may be substituted with 1 to 3 C₁₋₃ alkyl groups (e.g., methyl), k) a pyrimidinyl group, and l) an oxazolyl group,
   4) a pyrazolyl group which may be substituted with 1 to 3 substituents selected from a) a C₁₋₃ alkyl group (e.g., methyl, ethyl, isopropyl) which may be substituted with 1 to 3 halogen atoms (e.g., fluorine atom), b) a C₁₋₃ alkoxy group (e.g., methoxy) which may be substituted with 1 to 3 halogen atoms (e.g., fluorine atom), c) a cyano group, and d) a halogen atom (e.g., chlorine atom), or
   5) an imidazopyridyl group which may be substituted with 1 to 3 halogen atoms (e.g., chlorine atom).

B is preferably a pyridyl group which may be substituted with 1 to 3 substituents selected from a) a halogen atom (e.g., chlorine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy) which may be substituted with 1 to 3 halogen atoms (e.g., fluorine atom), and e) a triazolyl group.

Suitable examples of the compound (I) include the following compounds. The following suitable [Compound I-1] to [Compound I-9] include salts thereof, or co-crystals, hydrates or solvates thereof.

### [Compound I-1]

The compound (I), wherein A is 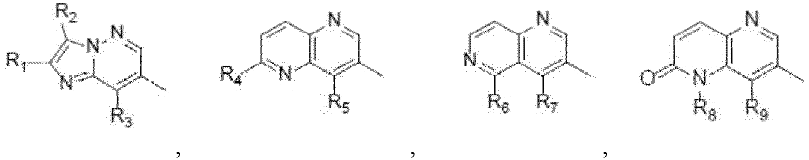 , or 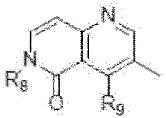
R₁ is 1) a hydrogen atom, 2) a halogen atom (e.g., chlorine atom, bromine atom), 3) a cyano group, 4) a C₁₋₃ alkyl group (e.g., methyl) which may be substituted with 1 to 3 halogen atoms (e.g., fluorine atom), 5) a C₁₋₃ alkoxy group (e.g., methoxy), 6) a C₃₋₆ cycloalkyl group (e.g., cyclopropyl), or 7) a phenyl group;
R₂ is 1) a hydrogen atom or 2) a halogen atom (e.g., fluorine atom, chlorine atom);
R₃ is 1) a C₁₋₆ alkyl group (e.g., ethyl, isopropyl, sec-butyl) which may be substituted with 1 to 3 substituents selected from a C₁₋₃ alkoxy group (e.g., methoxy), a hydroxyl group and a halogen atom (e.g., fluorine atom), 2) a pyrazolyl group (e.g., 4-pyrazolyl) which may be substituted with 1 to 3 substituents selected from a C₁₋₃ alkyl group (e.g., methyl) and a halogen atom (e.g., chlorine atom), 3) a C₃₋₆ cycloalkyl group (e.g., cyclopropyl), 4) an amino group di-substituted with a C₁₋₃ alkyl group (e.g., methyl), or 5) a phenyl group which may be substituted with 1 to 3 halogen atoms (e.g., chlorine atom);
R₄ is 1) a hydrogen atom, 2) a halogen atom (e.g., chlorine atom), 3) a C₁₋₃ alkyl group (e.g., methyl, ethyl) which may be substituted with 1 to 3 substituents selected from a) a hydroxyl group, b) a C₁₋₃ alkoxy group (e.g., methoxy) which may be substituted with a 4-methoxyphenyl group and c) a halogen atom (e.g., fluorine atom), or 4) a C₁₋₃ alkoxy group (e.g., methoxy, ethoxy) which may be substituted with 1 to 3 halogen atoms (e.g., fluorine atom);
R₅ is 1) a C₁₋₆ alkyl group (e.g., ethyl, isopropyl) which may be substituted with 1 to 3 C₁₋₃ alkoxy groups (e.g., methoxy, ethoxy) or 2) a phenyl group which may be substituted with 1 to 3 halogen atoms (e.g., chlorine atom);
R₆ is 1) a hydrogen atom, 2) a halogen atom (e.g., chlorine atom), 3) a C₁₋₃ alkyl group (e.g., methyl) or 4) a C₁₋₃ alkoxy group (e.g., ethoxy);
R₇ is 1) a C₁₋₆ alkyl group (e.g., ethyl, isopropyl) which may be substituted with 1 to 3 C₁₋₃ alkoxy groups (e.g., methoxy) or 2) a phenyl group which may be substituted with 1 to 3 halogen atoms (e.g., chlorine atom);
R₈ is a C₁₋₃ alkyl group (e.g., methyl);
R₉ is a C₁₋₃ alkyl group (e.g., ethyl) which may be substituted with 1 to 3 C₁₋₃ alkoxy groups (e.g., methoxy); and
B is
   1) a phenyl group which may be substituted with 1 to 3 substituents selected from a) a halogen atom (e.g., fluorine atom, chlorine atom), b) a cyano group, c) a C₁₋₃ alkoxy group (e.g., methoxy) which may be substituted with 1 to 3 halogen atoms (e.g., fluorine atom), and d) a triazolyl group,
   2) a C₃₋₆ cycloalkyl group (e.g., cyclohexyl) which may be substituted with 1 to 3 substituents selected from a) a C₁₋₃ alkyl group (e.g., methyl) which may be substituted with 1 to 3 halogen atoms (e.g., fluorine atom) and b) a halogen atom (e.g., fluorine atom),
   3) a pyridyl group which may be substituted with 1 to 3 substituents selected from a) a halogen atom (e.g., fluorine atom, chlorine atom, bromine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy, ethoxy) which may be substituted with 1 to 3 substituents selected from a halogen atom (e.g., fluorine atom) and a C₁₋₃ alkoxy group (e.g., methoxy), e) a pyrazolyl group which may be substituted with 1 to 3 C₁₋₃ alkyl groups (e.g., methyl), f) an imidazolyl group which may be substituted with 1 to 3 C₁₋₃ alkyl groups (e.g., methyl), g) a triazolyl group which may be substituted with 1 to 3 C₁₋₃ alkyl groups (e.g., methyl) which may be substituted with 1 to 3 substituents selected from a C₁₋₃ alkoxy group (e.g., methoxy) and a halogen atom (e.g., fluorine atom), h) an azetidinyl group, i) a pyrrolidonyl group, j) a tetrazolyl group which may be substituted with 1 to 3 C₁₋₃ alkyl groups (e.g., methyl), k) a pyrimidinyl group, and l) an oxazolyl group,
   4) a pyrazolyl group which may be substituted with 1 to 3 substituents selected from a) a C₁₋₃ alkyl group (e.g., methyl, ethyl, isopropyl) which may be substituted with 1 to 3 halogen atoms (e.g., fluorine atom), b) a C₁₋₃ alkoxy group (e.g., methoxy) which may be substituted with 1 to 3 halogen atoms (e.g., fluorine atom), c) a cyano group, and d) a halogen atom (e.g., chlorine atom), or
   5) an imidazopyridyl group which may be substituted with 1 to 3 halogen atoms (e.g., chlorine atom).

### [Compound I-2]

The compound (I), wherein A is 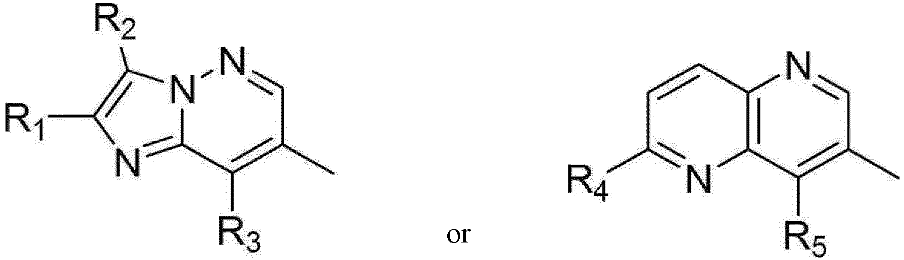
R₁ is 1) a halogen atom (e.g., chlorine atom) or 2) a C₁₋₃ alkyl group (e.g., methyl);
R₂ is a hydrogen atom;
R₃ is a C₁₋₆ alkyl group (e.g., ethyl, isopropyl) which may be substituted with 1 to 3 C₁₋₃ alkoxy groups (e.g., methoxy);
R₄ is 1) a halogen atom (e.g., chlorine atom) or 2) a C₁₋₃ alkyl group (e.g., methyl);
R₅ is a C₁₋₆ alkyl group (e.g., ethyl) which may be substituted with 1 to 3 C₁₋₃ alkoxy groups (e.g., methoxy); and
B is a pyridyl group which may be substituted with 1 to 3 substituents selected from a) a halogen atom (e.g., chlorine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy) which may be substituted with 1 to 3 halogen atoms (e.g., fluorine atom), and e) a triazolyl group.

### [Compound I-3]

The compound (I), wherein A is 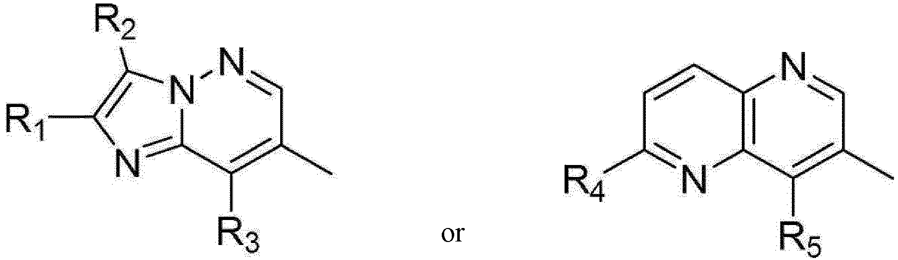
R₁ is a C₁₋₃ alkyl group (e.g., methyl);
R₂ is a hydrogen atom;
R₃ is a C₁₋₆ alkyl group (e.g., ethyl, isopropyl) which is substituted with 1 to 3 C₁₋₃ alkoxy groups (e.g., methoxy);
R₄ is 1) a halogen atom (e.g., chlorine atom) or 2) a C₁₋₃ alkyl group (e.g., methyl);
R₅ is a C₁₋₆ alkyl group (e.g., ethyl) which may be substituted with 1 to 3 C₁₋₃ alkoxy groups (e.g., methoxy); and
B is a pyridyl group which may be substituted with 1 to 3 substituents selected from a) a halogen atom (e.g., chlorine atom), b) a C₁₋₃ alkyl group (e.g., methyl) which may be substituted with 1 to 3 halogen atoms (e.g., fluorine atom), c) a C₁₋₃ alkoxy group (e.g., methoxy) which may be substituted with 1 to 3 halogen atoms (e.g., fluorine atom), and d) a triazolyl group.

### [Compound 1-4]

The compound (I), wherein A is 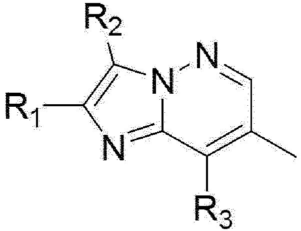
R₁ is 1) a hydrogen atom, 2) a halogen atom (e.g., chlorine atom, bromine atom), 3) a cyano group, 4) a C₁₋₃ alkyl group (e.g., methyl) which may be substituted with 1 to 3 halogen atoms (e.g., fluorine atom), 5) a C₁₋₃ alkoxy group (e.g., methoxy), 6) a C₃₋₆ cycloalkyl group (e.g., cyclopropyl), or 7) a phenyl group;
R₂ is 1) a hydrogen atom or 2) a halogen atom (e.g., fluorine atom, chlorine atom);
R₃ is 1) a C₁₋₆ alkyl group (e.g., ethyl, isopropyl, sec-butyl) which may be substituted with 1 to 3 substituents selected from a C₁₋₃ alkoxy group (e.g., methoxy), a hydroxyl group and a halogen atom (e.g., fluorine atom), 2) a pyrazolyl group (e.g., 4-pyrazolyl) which may be substituted with 1 to 3 substituents selected from a C₁₋₃ alkyl group (e.g., methyl) and a halogen atom (e.g., a chlorine atom), 3) a C₃₋₆ cycloalkyl group (e.g., cyclopropyl), 4) an amino group di-substituted with a C₁₋₃ alkyl group (e.g., methyl), or 5) a phenyl group which may be substituted with 1 to 3 halogen atoms (e.g., chlorine atom); and
B is
   1) a phenyl group which may be substituted with 1 to 3 substituents selected from a) a halogen atom (e.g., fluorine atom, chlorine atom), b) a cyano group, c) a C₁₋₃ alkoxy group (e.g., methoxy) which may be substituted with 1 to 3 halogen atoms (e.g., fluorine atom), and d) a triazolyl group,
   2) a pyridyl group which may be substituted with 1 to 3 substituents selected from a) a halogen atom (e.g., chlorine atom, bromine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy) which may be substituted with 1 to 3 halogen atoms (e.g., fluorine atom), e) a pyrazolyl group which may be substituted with 1 to 3 C₁₋₃ alkyl groups (e.g., methyl), f) an imidazolyl group which may be substituted with 1 to 3 C₁₋₃ alkyl groups (e.g., methyl), g) a triazolyl group which may be substituted with 1 to 3 C₁₋₃ alkyl groups (e.g., methyl) which may be substituted with 1 to 3 substituents selected from a C₁₋₃ alkoxy group (e.g., methoxy) and a halogen atom (e.g., fluorine atom), and h) an oxazolyl group,
   3) a pyrazolyl group which may be substituted with 1 to 3 substituents selected from a) a C₁₋₃ alkyl group (e.g., methyl) which may be substituted with 1 to 3 halogen atoms (e.g., fluorine atom), b) a C₁₋₃ alkoxy group (e.g., methoxy) which may be substituted with 1 to 3 halogen atoms (e.g., fluorine atom), c) a cyano group, and d) a halogen atom (e.g., chlorine atom), or
   4) an imidazopyridyl group which may be substituted with 1 to 3 halogen atoms (e.g., chlorine atom).

### [Compound I-5]

The compound (I), wherein A is 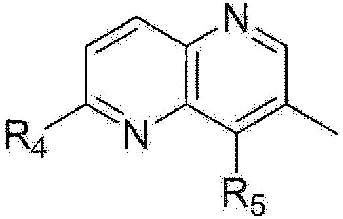
R₄ is 1) a hydrogen atom, 2) a halogen atom (e.g., chlorine atom), 3) a C₁₋₃ alkyl group (e.g., methyl, ethyl) which may be substituted with 1 to 3 substituents selected from a) a hydroxyl group, b) a C₁₋₃ alkoxy group (e.g., methoxy) which may be substituted with a 4-methoxyphenyl group and c) a halogen atom (e.g., fluorine atom), or 4) a C₁₋₃ alkoxy group (e.g., methoxy, ethoxy) which may be substituted with 1 to 3 halogen atoms (e.g., fluorine atom);
R₅ is 1) a C₁₋₆ alkyl group (e.g., ethyl, isopropyl) which may be substituted with 1 to 3 C₁₋₃ alkoxy groups (e.g., methoxy, ethoxy) or 2) a phenyl group which may be substituted with 1 to 3 halogen atoms (e.g., chlorine atom); and
B is
   1) a phenyl group which may be substituted with 1 to 3 halogen atoms (e.g., fluorine atom),
   2) a C₃₋₆ cycloalkyl group (e.g., cyclohexyl) which may be substituted with 1 to 3 substituents selected from a) a C₁₋₃ alkyl group (e.g., methyl) which may be substituted with 1 to 3 halogen atoms (e.g., fluorine atom) and b) a halogen atom (e.g., fluorine atom),
   3) a pyridyl group which may be substituted with 1 to 3 substituents selected from a) a halogen atom (e.g., fluorine atom, chlorine atom, bromine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy, ethoxy) which may be substituted with 1 to 3 substituents selected from a halogen atom (e.g., fluorine atom) and a C₁₋₃ alkoxy group (e.g., methoxy), e) a triazolyl group which may be substituted with 1 to 3 C₁₋₃ alkyl groups (e.g., methyl) which may be substituted with 1 to 3 substituents selected from a C₁₋₃ alkoxy group (e.g., methoxy) and a halogen atom (e.g., fluorine atom), f) an azetidinyl group, g) a pyrrolidonyl group, h) a tetrazolyl group which may be substituted with 1 to 3 substituents selected from a C₁₋₃ alkyl group (e.g., methyl), i) a pyrimidinyl group, and j) an oxazolyl group, or
   4) a pyrazolyl group which may be substituted with 1 to 3 C₁₋₃ alkyl groups (e.g., methyl, ethyl, isopropyl) which may be substituted with 1 to 3 halogen atoms (e.g., fluorine atom).

### [Compound I-6]

The compound (I), wherein A is 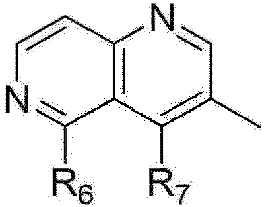
R₆ is 1) a hydrogen atom, 2) a halogen atom (e.g., chlorine atom), 3) a C₁₋₃ alkyl group (e.g., methyl) or 4) a C₁₋₃ alkoxy group (e.g., ethoxy);
R₇ is 1) a C₁₋₆ alkyl group (e.g., ethyl, isopropyl) which may be substituted with 1 to 3 C₁₋₃ alkoxy groups (e.g., methoxy) or 2) a phenyl group which may be substituted with 1 to 3 halogen atoms (e.g., chlorine atom); and
B is
   1) a phenyl group which may be substituted with 1 to 3 halogen atoms (e.g., fluorine atom), or
   2) a pyridyl group which may be substituted with 1 to 3 substituents selected from a) a halogen atom (e.g., chlorine atom), b) a cyano group, c) a C₁₋₃ alkyl group (e.g., methyl) which may be substituted with 1 to 3 halogen atoms (e.g., fluorine atom), d) a C₁₋₃ alkoxy group (e.g., methoxy) which may be substituted with 1 to 3 halogen atoms (e.g., fluorine atom), and e) a triazolyl group.

### [Compound I-7]

The compound (I), wherein A is 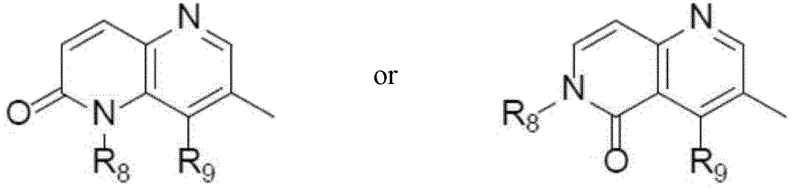
R₈ is a C₁₋₃ alkyl group (e.g., methyl);
R₉ is a C₁₋₃ alkyl group (e.g., ethyl) which may be substituted with 1 to 3 C₁₋₃ alkoxy groups (e.g., methoxy); and
B is a pyridyl group which may be substituted with 1 to 3 substituents selected from a) a halogen atom (e.g., chlorine atom), b) a C₁₋₃ alkyl group (e.g., methyl) which may be substituted with 1 to 3 halogen atoms (e.g., fluorine atom), c) a C₁₋₃ alkoxy group (e.g., methoxy), and d) a triazolyl group.

### [Compound I-8]

(S)-N-(5-chloro-6-(difluoromethoxy)pyridin-3-yl)-N'-(8-(1-methoxyethyl)-2-methylimidazo[1,2-b]pyridazin-7-yl)urea,
(S)-N-(6-chloro-4-(1-methoxyethyl)-1,5-naphthyridin-3-yl)-N'-(6-(2H-1,2,3-triazol-2-yl)-5-(trifluoromethyl)pyridin-3-yl)urea,
(S)-N-(4-(1-methoxyethyl)-6-methyl-1,5-naphthyridin-3-yl)-N'-(6-(2H-1,2,3-triazol-2-yl)-5-(trifluoromethyl)pyridin-3-yl)urea,
(S)-N-(5-chloro-6-(2H-1,2,3-triazol-2-yl)pyridin-3-yl)-N'-(8-(1-methoxyethyl)-2-methylimidazo[1,2-b]pyridazin-7-yl)urea,
(S)-N-(5-cyano-6-(difluoromethoxy)pyridin-3-yl)-N'-(8-(1-methoxyethyl)-2-methylimidazo[1,2-b]pyridazin-7-yl)urea,
(S)-N-(8-(1-methoxyethyl)-2-methylimidazo[1,2-b]pyridazin-7-yl)-N'-(6-(2H-1,2,3-triazol-2-yl)-5-(trifluoromethyl)pyridin-3-yl)urea,
N-(5-chloro-6-(2H-1,2,3-triazol-2-yl)pyridin-3-yl)-N'-(8-(2-methoxypropan-2-yl)-2-methylimidazo[1,2-b]pyridazin-7-yl)urea,
N-(5-chloro-6-(difluoromethoxy)pyridin-3-yl)-N'-(2-chloro-8-(propan-2-yl)imidazo[1,2-b]pyridazin-7-yl)urea, or
N-(5-chloro-6-(2H-1,2,3-triazol-2-yl)pyridin-3-yl)-N'-(2-methyl-8-(propan-2-yl)imidazo[1,2-b]pyridazin-7-yl)urea.

### [Compound I-9]

(S)-N-(5-chloro-6-(difluoromethoxy)pyridin-3-yl)-N'-(8-(1-methoxyethyl)-2-methylimidazo[1,2-b]pyridazin-7-yl)urea, or
(S)-N-(6-chloro-4-(1-methoxyethyl)-1,5-naphthyridin-3-yl)-N'-(6-(2H-1,2,3-triazol-2-yl)-5-(trifluoromethyl)pyridin-3-yl)urea (sometimes abbreviated as Compound A in the description).

The salt of the compound represented by the formula (I) is preferably a pharmacologically acceptable salt. Examples of such a salt include salts with inorganic bases, salts with organic bases, salts with inorganic acids, salts with organic acids, and salts with basic or acidic amino acids.

Suitable examples of the salts with inorganic bases include alkali metal salts such as sodium salt and potassium salt; alkaline earth metal salts such as calcium salt and magnesium salt; aluminum salt; and ammonium salt.

Suitable examples of the salts with organic bases include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine and N,N-dibenzylethylenediamine.

Suitable examples of the salts with inorganic acids include salts with hydrogen chloride, hydrogen bromide, nitric acid, sulfuric acid and phosphoric acid.

Suitable examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid.

Suitable examples of the salts with basic amino acids include salts with arginine, lysine and ornithine.

Suitable examples of the salts with acidic amino acids include salts with aspartic acid and glutamic acid.

The compound (I) can be produced according to the method described in Patent Literature 1 (for example, Reference Examples 1 to 4, Examples 1 to 9, and the like) or the method described in WO2021/241611 A.

When the compound (I) contains optical isomers, steric isomers, positional isomers and rotational isomers, these isomers are also included as the compound (I), and can be each obtained as a single product by a synthesis technique and a separating technique known per se. For example, when the compound (I) has an optical isomer, the optical isomer separated from the compound is also included in the compound (I).

Here, the optical isomer can be produced by a method known per se.

The compound (I) may be crystalline.

The crystal of the compound (I) (hereinafter, sometimes abbreviated as "crystal of the present invention") can be produced by applying a crystallization method known per se to the compound (I) to crystallize the same.

The crystal of the present invention is expected to have excellent physicochemical properties (e.g., melting point, solubility, stability) and biological properties (e.g., in vivo kinetics (absorption, distribution, metabolism, excretion), efficacy of medicine) and be useful as a medicine.

The compound (I) may be a pharmaceutically acceptable co-crystal or co-crystal salt. Here, the co-crystal or co-crystal salt means a crystalline substance composed of two or more unique solids at room temperature, which solids are different in physical property (e.g., structure, melting point, heat of fusion, hygroscopicity, solubility and stability) from each other. The co-crystal or co-crystal salt can be produced according to a co-crystallization method known per se.

The compound (I) may be a hydrate, a non-hydrate, a non-solvate or a solvate.

Furthermore, a deuterium-converted compound in which ¹H has been converted to ²H (D) is also included in the compound (I).

The compound (I) may be labeled with an isotope (e.g., ³H, ¹³C, ¹⁴C, ¹⁸F, ³⁵S, ¹²⁵I) or the like. The isotope-labeled or substituted compound (I) can be used, for example, as a tracer (PET (positron emission tomography) tracer) used in PET, and is expected as useful in fields such as medical diagnosis.

The compound (I) may be used as a prodrug.

The prodrug of the compound (I) is a compound that is converted to the compound (I) by a reaction with an enzyme, gastric acid or the like under physiological conditions in the living body, that is, a compound that enzymatically undergoes oxidation, reduction, hydrolysis etc. to change to the compound (I), or a compound that undergoes hydrolysis or the like due to gastric acid or the like to change to the compound (I).

The prodrug of the compound (I) includes a compound having the amino group of the compound (I) acylated, alkylated or phosphorylated (e.g., a compound having the amino group of the compound (I) eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated); a compound having the hydroxy group of the compound (I) acylated, alkylated, phosphorylated or borated (e.g., a compound having the hydroxy group of the compound (I) acetylated, palmitoylated, propanoylated, pivaloylated, succinylated, fumarylated, alanylated, or dimethylaminomethylcarbonylated); a compound having the carboxy group of the compound (I) esterified or amidated (e.g., a compound having the carboxy group of the compound (I) ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, or methylamidated). These compounds can be produced from the compound (I) by a method known per se.

In addition, the prodrug of the compound (I) may be one changing to the compound (I) under physiological conditions, as described in Molecular Design, pp. 163 to 198 in "Pharmaceutical research and development", Vol. 7, by Hirokawa Shoten in 1990.

In the present disclosure, the prodrug may form a salt. Such a salt includes those exemplified as the salt of the compound represented by the above formula (I).

The MALT1 inhibitor used in the combination of the present invention can be orally or parenterally administered to a mammal (preferably human) as a medicine, either as it is or in combination with a pharmacologically acceptable carrier.

The dosage form of the medicine can be appropriately selected according to the type and administration form of the "anticancer agent" to be combined, and for example, the medicine can be administered in the form of an oral preparation such as a tablet (e.g., a sugar-coated tablet, a film-coated tablet, a sublingual tablet, a buccal tablet, an orally fast-disintegrating tablet), for example, a parenteral preparation such as an injection or an infusion. Also, the medicine can be produced by a known producing method generally used in the pharmaceutical field (e.g., the method described in the Japanese Pharmacopoeia).

In the present disclosure, examples of the pharmacologically acceptable carrier include additives such as excipients, binders, disintegrants, lubricants, sweeteners, surfactants, suspending agents, emulsifiers, coloring agents, preservatives, aromatic substances, flavoring agents, stabilizers and thickeners commonly used in the pharmaceutical field.

Examples of the excipient include lactose, sucrose, glucose, starch, sucrose, microcrystalline cellulose, powdered glycyrrhiza, mannitol, sodium hydrogen carbonate, calcium phosphate and calcium sulfate.

Examples of the binder include 5 to 10% by weight starch paste, 10 to 20% by weight gum arabic solution or gelatin solution, 1 to 5% by weight tragacanth solution, carboxymethyl cellulose solution, sodium alginate solution or glycerin.

Examples of the disintegrant include starch and calcium carbonate.

Examples of the lubricant include magnesium stearate, stearic acid, calcium stearate and purified talc.

Examples of the sweetener include glucose, fructose, inverted sugar, sorbitol, xylitol, glycerin and simple syrup.

Examples of the surfactant include sodium lauryl sulfate, Polysorbate 80, sorbitan monofatty acid ester and Polyoxyl 40 stearate.

Examples of the suspending agent include gum arabic, sodium alginate, sodium carboxymethyl cellulose, methyl cellulose and bentonite.

Examples of the emulsifier include gum arabic, tragacanth, gelatin and Polysorbate 80.

The dosage of the MALT1 inhibitor used in the combination of the present invention varies depending on the type of the compound, age, body weight, symptom (patient condition), therapeutic effect, administration method, administration route, treatment time, and the like, but is adjusted so as to provide an optimum desired effect.

For example, when the daily dose of the MALT1 inhibitor used in the combination of the present invention is orally administered to a patient for the purpose of treating cancer, the daily dose for an adult (body weight of about 60 kg) is about 0.5 to about 2000 mg, preferably about 1 to about 1000 mg, more preferably about 3 to about 300 mg, and further preferably about 10 to about 200 mg, as the MALT1 inhibitor (for example, compound (I)). The daily dose may be administered in a single dose or in 2 to 3 divided doses.

### (2) Anticancer agent

In the present disclosure, the anticancer agent means an anticancer agent administered for the treatment of hematological cancer. The anticancer agent is not particularly limited as long as it is an anticancer agent administered for the treatment of hematological cancer, and examples thereof include cytotoxic anticancer agents, molecular targeted drugs, and chimeric antigen receptor (CAR)-expressing T cells (CAR-T cells).

In the present disclosure, the cytotoxic anticancer agent means an anticancer agent having an action of damaging cancer cells by inhibiting DNA synthesis, cell division, and the like of cancer cells.

Examples of the cytotoxic anticancer agent include alkylating agents, anticancer antibiotics, antimetabolites, plant alkaloids, immunomodulators, platinum preparations, and steroids.

Examples of the alkylating agents include Ifosfamide, Cyclophosphamide, Dacarbazine, Busulfan, Procarbazine, Bendamustine, Melphalan, Ranimustine, Carmustine, Chlorambucil, Lomustine, Mechlorethamine, Nimustine, Carbocone, and Thiotepa.

Examples of the anticancer antibiotics include Idarubicin, Epirubicin, Daunorubicin, Doxorubicin, Pirarubicin, Bleomycin, Mitoxantrone, Aclarubicin, Mitomycin C, Aclacinon, Zinostatin Stimalamer, Neocarzinostatin, and Pepleomycin.

Examples of the antimetabolites include Azacitidine, Enocitabine, Cladribine, Gemcitabine, Cytarabine, Thioguanine, Nelarabine, Hydroxyurea, Fludarabine, Pentostatin, Methotrexate, Pemetrexed, Mercaptopurine, Decitabine, Guadecitabine, CPX-351, Clofarabine, Hydroxycarbamide, Capecitabine, Carmofur, Tegafur, TS-1, Doxifluridine, and Fluorouracil.

Examples of the plant alkaloids include Irinotecan, Etoposide, Sobuzoxane, Vincristine, Vindesine, Vinblastine, Docetaxel, Nogitecan, Paclitaxel, and Vinorelbine.

Examples of the immunomodulators include Thalidomide, Lenalidomide, and Pomalidomide.

Examples of the platinum preparations include Carboplatin, Cisplatin, Nedaplatin, and Oxaliplatin.

Examples of the steroids include Prednisolone.

In the present disclosure, the cytotoxic anticancer agent preferably includes Cyclophosphamide, Doxorubicin, Vincristine, Bendamustine, Irinotecan, Prednisolone, and Lenalidomide.

In the present disclosure, the molecular targeted drug means an anticancer agent that targets a molecule related to proliferation, invasion, and metastasis of cancer cells.

Examples of the molecular targeted drug used for treatment of hematological cancer include inhibitors of a molecule selected from the group consisting of CD20, CD30, CD33, CD38, CD47, CD52, CD70, CD79, PD-1, PD-L1, ABL, AXL, proteasome, CCR4, JAK, CXCR4, BET, Bcl-2, HDAC, FLT3, LSD1, MDM2, IDH1, IDH2, Btk, IRAK4, PI3K, PKC, SYK, mTOR, Akt, MEK, EZH2, PLK, HSP90, XPO1, SMO, and NEDD8. Examples of the molecular targeted drug will be described below, but the molecular targeted drug is not limited thereto.

Examples of the molecular targeted drug used for treatment of hematological cancer include anti-CD20 antibodies, anti-CD30 antibody-drug conjugates, anti-CD33 antibodies, anti-CD38 antibodies, anti-CD47 antibodies, anti-CD52 antibodies, anti-CD70 antibodies, anti-CD79 antibody-drug conjugates, anti-PD-1 antibodies, anti-PD-L1 antibodies, ABL inhibitors, AXL inhibitors, proteasome inhibitors, anti-CCR4 antibodies, JAK inhibitors, CXCR4 antagonists, BET inhibitors, Bcl-2 inhibitors, HDAC inhibitors, FLT3 inhibitors, LSD1 inhibitors, MDM2 inhibitors, IDH1 inhibitors, IDH2 inhibitors, Btk inhibitors, PI3K inhibitors, PKC inhibitors, SYK inhibitors, mTOR inhibitors, Akt inhibitors, MEK inhibitors, EZH2 inhibitors, PLK inhibitors, HSP90 inhibitors, XPO1 inhibitors, SMO inhibitors, NEDD8 inhibitors, Btk degradation inducers, IRAK4 degradation inducers, IRAK4 inhibitors, CDK4/6 inhibitors, and retinoids.

Examples of the anti-CD20 antibodies include Ofatumumab, Rituximab, Ublituximab, and Obinutuzumab.

Examples of the anti-CD30 antibody-drug conjugates include Brentuximab Vedotin.

Examples of the anti-CD33 antibodies include Gemtuzumab and Gemtuzumab ozogamicin.

Examples of the anti-CD38 antibodies include Daratumumab and Isatuximab.

Examples of the anti-CD52 antibodies include Alemtuzumab.

Examples of the anti-CD70 antibodies include Cusatuzumab.

Examples of the anti-CD79 antibody-drug conjugates include Polatuzumab vedotin.

Examples of the ABL inhibitors include Imatinib, Dasatinib, and Nilotinib.

Examples of the AXL inhibitors include ONO-7475 and Bemcentinib.

Examples of the proteasome inhibitors include Bortezomib and Carfilzomib.

Examples of the anti-CCR4 antibodies include Mogamulizumab.

Examples of the JAK inhibitors include Ruxolitinib.

Examples of the CXCR4 antagonists include AMD3100, BMS-936564, BL-8040, Dociparstat sodium, and LY2624587 (CXCR4 inhibiting antibody).

Examples of the BET inhibitors include OTX015, GSK525762, RVX-208, BMS-986158, PLX51107, CPI-0610, TEN-010, INCB054329, ABBV075, and GS-5829.

Examples of the Bcl-2 inhibitors include Venetoclax, ABT-263, GX15-070, and AT-101.

Examples of the HDAC inhibitors include Tucidinostat, Pracinostat, Vorinostat, Romidepsin, Panobinostat, Belinostat, Entinostat, and Chidamide.

Examples of the FLT3 inhibitors include Gilteritinib, Midostaurin, Sorafenib, Ponatinib, Crenolanib, Tandutinib, Sunitinib, Quizartinib, and Pacritinib.

Examples of the LSD1 inhibitors include GSK-2879552, INCB-59872, and ORY-1001.

Examples of the MDM2 inhibitors include Idasanutlin, SAR405838, DS-3032b, RG7112, HDM201, MK4828, AMG-232, and ALRN-6924.

Examples of the IDH1 inhibitors include Ivosidenib.

Examples of the IDH2 inhibitors include Enasidenib.

Examples of the Btk inhibitors include Ibrutinib, Tirabrutinib, Spebrutinib, Acarabrutinib, Evobrutinib, Fenebrutinib, Posebrutinib, Vecabrutinib, Zanubrutinib, Branebrutinib, PRN-1008, BMS-986142, LOU-064, M-7583, AC-058, DTRMWXHS-12, TAS-5315, TAK-020, ARQ-531, BMS-935177, PCI-45292, PRN-2246, SHR-1459, ABBV-105, CT-1530, ICP022, LOXO-305, JNJ-64264681, and HWH-486.

Examples of the PI3K inhibitors include Idelalisib, Duvelisib, Alpelisib, Copanlisib, Umbralisib, Pictilisib, Buparlisib, Dactolisib, Pilaralisib, Taselisib, PX866, CH5132799, ZSTK474, SF1126, MLN1117, Apitolisib, Gedatolisib, Paxalisib, Bimiralisib, Samotolisib, Voxtalisib, BKM120, GDC-0941, and Zandelisib.

Examples of the PKC inhibitors include Enzastaurin, Sotrastaurin, Ruboxistaurin, Staurosporine, and Midostaurin.

Examples of the SYK inhibitors include Fostamatinib, Entospletinib, Cerdulatinib, GS-9876, TAK-659, and PRT062607.

Examples of the mTOR inhibitors include Sirolimus, Temsirolimus, Everolimus, Ridaforolimus, Sapanisertib, Vistusertib, MLN0128, CC-223, and RapaLink-1.

Examples of the Akt inhibitors include MK-2206, TAS-117, Uprosertib, Capivasertib, and Ipatasertib.

Examples of the MEK inhibitors include Trametinib, Selumetinib, and Cobimetinib.

Examples of the EZH2 inhibitors include Tazemetostat.

Examples of the PLK inhibitors include Volasertib, GSK461364, Rigosertib, BI2536, HMN-176, NMS-P937, CYC-140, and RO3280.

Examples of the HSP90 inhibitors include Ganetespib.

Examples of the XPO1 inhibitor include Selinexor.

Examples of the SMO inhibitors include Glasdegib, Vismodegib, and Sonidegib.

Examples of the NEDD8 inhibitors include Pevonedistat.

Examples of the Btk degradation inducers include NX-2127, NX-5948, and BGB-16673.

Examples of the IRAK4 inhibitors include CA-4948, GS-5718, BAY-1834845, and PF-06650833.

Examples of the CDK4/6 inhibitors include Palbociclib, Abemaciclib, Ribociclib, and Dinaciclib.

Examples of the retinoid include Bexarotene.

In the present disclosure, the chimeric antigen receptor (CAR)-expressing T cell means a T cell expressing a chimeric antigen receptor (CAR) (CAR-T cell) that targets a molecule expressed on the surface of a cancer cell.

Examples of the CAR-T cell include Tisagenlecleucel, Axicabtagene ciloleucel, Brexucabtagene autoleucel (KTE-X19), Lisocabtagene maraleucel, and Idecabtagene vicleucel.

Any one or any two or more of these anticancer agents can be used in hematological cancer treatment in combination with the MALT1 inhibitor according to the present invention.

The dosage of the anticancer agent used in the combination of the present invention varies depending on age, body weight, symptom, therapeutic effect, administration method, treatment time, and the like, but is adjusted so as to provide an optimum desired effect. When the anticancer agent used is already clinically applied, a clinical dose can be referred to.

The anticancer agent used in the combination of the present invention can be orally or parenterally administered to a mammal (preferably human) as a medicine, either as it is or in combination with a pharmacologically acceptable carrier.

The dosage form of the medicine can be appropriately selected according to the type and administration form of the "MALT1 inhibitor" to be combined, and the medicine can be administered in the form of an oral preparation such as a tablet (e.g., a sugar-coated tablet, a film-coated tablet, a sublingual tablet, a buccal tablet, an orally fast-disintegrating tablet), or a parenteral preparation such as an injection or an infusion. Also, the medicine can be produced by a known producing method generally used in the pharmaceutical field (e.g., the method described in the Japanese Pharmacopoeia). For more specific aspects, those described above for the MALT1 inhibitor can be referred to. When the anticancer agent used is already clinically applied, a dosage form thereof may be referred to.

### [Toxicity]

The combination of the present invention has sufficiently low toxicity and thus can be used safely as a drug.

### [Drug Applications]

The hematological cancer to be treated with the combination of the present invention or the compound (I) which is a MALT1 inhibitor is not particularly limited, and examples thereof include multiple myeloma, leukemia (for example, acute myeloid leukemia, acute lymphocytic leukemia, chronic lymphocytic leukemia), malignant lymphoma (for example, B-cell lymphoma (for example, Burkitt lymphoma, AIDS-related lymphoma, marginal zone B-cell lymphoma, diffuse large B-cell lymphoma, primary effusion lymphoma, lymphomatoid granulomatosis, follicular lymphoma, B-cell chronic lymphocytic leukemia, B-cell prolymphocytic leukemia, lymphoplasmacytic leukemia/Waldenstrom macroglobulinemia, plasmacytoma, mantle cell lymphoma, mediastinal large B-cell lymphoma, intravascular large B-cell lymphoma, hairy cell leukemia), T-cell lymphoma (for example, adult T-cell leukemia/lymphoma, cutaneous T-cell lymphoma, peripheral T-cell lymphoma, angioimmunoblastic T-cell lymphoma, anaplastic large cell lymphoma, extranodal NK/T-cell lymphoma), chronic myeloproliferative disease, and the like), Hodgkin disease, and cancer of unknow primary.

As one aspect, the combination of the present invention is also applicable to treatment of metastatic cancer and suppression of metastasis.

As one aspect, the combination of the present invention suppresses recurrence.

In the present invention, treatment is meant to produce the effect of at least one of prolonging progression-free survival (PFS), prolonging overall survival (OS), prolonging disease-free survival (DFS), prolonging time to progression (TTP), prolonging event-free survival (EFS), prolonging recurrence-free survival (RFS), decreasing the number of cancer cells, decreasing tumor size, suppressing (delaying or stopping) tumor growth, suppressing (delaying or stopping) tumor metastasis, suppressing (preventing or delaying) recurrence, and ameliorating one or more symptoms associated with cancer.

In the present disclosure, the phrase "administered in combination" includes simultaneous administration of compounds in a same preparation or separate preparations and separate administration of compounds (for example, sequential administration). More specifically, it may be administered in the form of a combined agent containing all components in one preparation, or may be administered in the form of separate preparations. When administered as separate preparations, simultaneous administration and staggered administration are included. Further, for the staggered administration, the MALT1 inhibitor may be administered first and the anticancer agent may be administered later, or the anticancer agent may be administered first and the MALT1 inhibitor may be administered later. Each administration method may be the same or different.

In the present disclosure, the combination of the present invention may be administered in combination with another drug (for example, known cancer therapeutic agent) for (1) complementation and/or enhancement of a therapeutic effect, (2) improvement in kinetics and absorption, reduction in dosage, and/or (3) reduction in side effects.

In the combination of the present invention, the blending ratio of the MALT1 inhibitor and the anticancer agent can be appropriately selected depending on the subject to be administered, administration route, target disease, symptom, specific combination of drugs, and the like. For example, when the subject to be administered is a human, 0.01 to 100 parts by weight of the anticancer agent may be used with respect to 1 part by weight of the MALT1 inhibitor.

The present application provides, for example, the following embodiments.
[1] A therapeutic agent against hematological cancer, wherein the agent is to be administered in combination with a cytotoxic anticancer agent and/or a molecular targeted drug, comprising a compound represented by a formula (I): 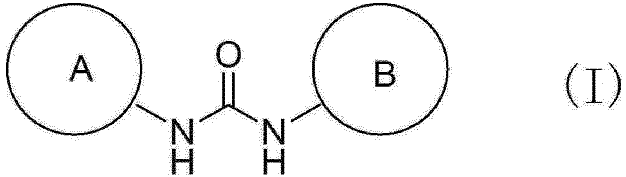 (wherein
   A represents 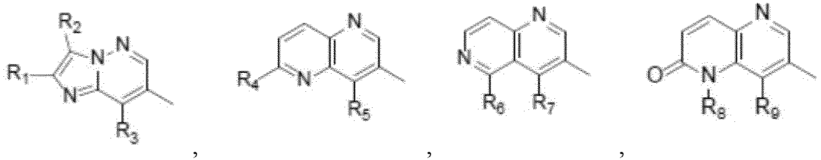 , or 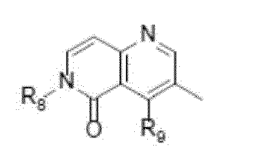
   R₁ represents 1) a hydrogen atom, 2) a halogen atom, 3) a cyano group, 4) a C₁₋₃ alkyl group which may be substituted with 1 to 3 halogen atoms, 5) a C₁₋₃ alkoxy group, 6) a C₃₋₆ cycloalkyl group, or 7) a phenyl group;
   R₂ represents 1) a hydrogen atom or 2) a halogen atom;
   R₃ represents 1) a C₁₋₆ alkyl group which may be substituted with 1 to 3 substituents selected from a C₁₋₃ alkoxy group, a hydroxyl group and a halogen atom, 2) a pyrazolyl group which may be substituted with 1 to 3 substituents selected from a C₁₋₃ alkyl group and a halogen atom, 3) a C₃₋₆ cycloalkyl group, 4) an amino group di-substituted with a C₁₋₃ alkyl group, or 5) a phenyl group which may be substituted with 1 to 3 halogen atoms;
   R₄ and R₆ represent 1) a hydrogen atom, 2) a halogen atom, 3) a C₁₋₃ alkyl group which may be substituted with 1 to 3 substituents selected from a) a hydroxyl group, b) a C₁₋₃ alkoxy group which may be substituted with a 4-methoxyphenyl group and c) a halogen atom, or 4) a C₁₋₃ alkoxy group which may be substituted with 1 to 3 halogen atoms;
   R₅, R₇ and R₉ represent 1) a C₁₋₆ alkyl group which may be substituted with 1 to 3 C₁₋₃ alkoxy groups or 2) a phenyl group which may be substituted with 1 to 3 halogen atoms;
   R₈ represents a C₁₋₃ alkyl group; and
   B represents
      1) a phenyl group which may be substituted with 1 to 3 substituents selected from a) a halogen atom, b) a cyano group, c) a C₁₋₃ alkoxy group which may be substituted with 1 to 3 halogen atoms, and d) a triazolyl group,
      2) a C₃₋₆ cycloalkyl group which may be substituted with 1 to 3 substituents selected from a) a C₁₋₃ alkyl group which may be substituted with 1 to 3 halogen atoms and b) a halogen atom,
      3) a pyridyl group which may be substituted with 1 to 3 substituents selected from a) a halogen atom, b) a cyano group, c) a C₁₋₃ alkyl group which may be substituted with 1 to 3 halogen atoms, d) a C₁₋₃ alkoxy group which may be substituted with 1 to 3 substituents selected from a halogen atom and a C₁₋₃ alkoxy group, e) a pyrazolyl group which may be substituted with 1 to 3 C₁₋₃ alkyl groups, f) an imidazolyl group which may be substituted with 1 to 3 C₁₋₃ alkyl groups, g) a triazolyl group which may be substituted with 1 to 3 C₁₋₃ alkyl groups which may be substituted with 1 to 3 substituents selected from a C₁₋₃ alkoxy group and a halogen atom, h) an azetidinyl group, i) a pyrrolidonyl group, j) a tetrazolyl group which may be substituted with 1 to 3 C₁₋₃ alkyl groups, k) a pyrimidinyl group, and l) an oxazolyl group,
      4) a pyrazolyl group which may be substituted with 1 to 3 substituents selected from a) a C₁₋₃ alkyl group which may be substituted with 1 to 3 halogen atoms, b) a C₁₋₃ alkoxy group which may be substituted with 1 to 3 halogen atoms, c) a cyano group, and d) a halogen atom, or
      5) an imidazopyridyl group which may be substituted with 1 to 3 halogen atoms)
      or a salt thereof, or a co-crystal, a hydrate or a solvate thereof.
[2] A therapeutic agent against hematological cancer comprising a cytotoxic anticancer agent and/or a molecular targeted drug, wherein the agent is to be administered in combination with the compound represented by the formula (I) or a salt thereof, or a co-crystal, a hydrate or a solvate thereof according to [1].
[3] The therapeutic agent against hematological cancer according to [1] or [2], wherein the compound represented by the formula (I) is:
   (1) (S)-N-(5-chloro-6-(difluoromethoxy)pyridin-3-yl)-N'-(8-(1-methoxyethyl)-2-methylimidazo[1,2-b]pyridazin-7-yl)urea,
   (2) (S)-N-(6-chloro-4-(1-methoxyethyl)-1,5-naphthyridin-3-yl)-N'-(6-(2H-1,2,3-triazol-2-yl)-5-(trifluoromethyl)pyridin-3-yl)urea,
   (3) (S)-N-(4-(1-methoxyethyl)-6-methyl-1,5-naphthyridin-3-yl)-N'-(6-(2H-1,2,3-triazol-2-yl)-5-(trifluoromethyl)pyridin-3-yl)urea,
   (4) (S)-N-(5-chloro-6-(2H-1,2,3-triazol-2-yl)pyridin-3-yl)-N'-(8-(1-methoxyethyl)-2-methylimidazo[1,2-b]pyridazin-7-yl)urea,
   (5) (S)-N-(5-cyano-6-(difluoromethoxy)pyridin-3-yl)-N'-(8-(1-methoxyethyl)-2-methylimidazo[1,2-b]pyridazin-7-yl)urea,
   (6) (S)-N-(8-(1-methoxyethyl)-2-methylimidazo[1,2-b]pyridazin-7-yl)-N'-(6-(2H-1,2,3-triazol-2-yl)-5-(trifluoromethyl)pyridin-3-yl)urea,
   (7) N-(5-chloro-6-(2H-1,2,3-triazol-2-yl)pyridin-3-yl)-N'-(8-(2-methoxypropan-2-yl)-2-methylimidazo[1,2-b]pyridazin-7-yl)urea,
   (8) N-(5-chloro-6-(difluoromethoxy)pyridin-3-yl)-N'-(2-chloro-8-(propan-2-yl)imidazo[1,2-b]pyridazin-7-yl)urea, or
   (9) N-(5-chloro-6-(2H-1,2,3-triazol-2-yl)pyridin-3-yl)-N'-(2-methyl-8-(propan-2-yl)imidazo[1,2-b]pyridazin-7-yl)urea.
[4] The therapeutic agent against hematological cancer according to any one of [1] to [3], wherein the compound represented by the formula (I) is (S)-N-(5-chloro-6-(difluoromethoxy)pyridin-3-yl)-N'-(8-(1-methoxyethyl)-2-methylimidazo[1,2-b]pyridazin-7-yl)urea.
[5] The therapeutic agent against hematological cancer according to any one of [1] to [3], wherein the compound represented by the formula (I) is (S)-N-(6-chloro-4-(1-methoxyethyl)-1, 5-naphthyridin-3-yl)-N'-(6-(2H-1,2,3-triazol-2-yl)-5-(trifluoromethyl)pyridin-3-yl)urea.
[6] The therapeutic agent against hematological cancer according to any one of [1] to [5], wherein the cytotoxic anticancer agent is one or more selected from the group consisting of Cyclophosphamide, Doxorubicin, Vincristine, Bendamustine, Irinotecan, Prednisolone, and Lenalidomide.
[7] The therapeutic agent against hematological cancer according to any one of [1] to [5], wherein the molecular targeted drug is one or more selected from the group consisting of an anti-CD20 antibody, an anti-CD30 antibody-drug conjugate, an anti-CD33 antibody, an anti-CD38 antibody, an anti-CD47 antibody, an anti-CD52 antibody, an anti-CD70 antibody, an anti-CD79 antibody-drug conjugate, an anti-PD-1 antibody, an anti-PD-L1 antibody, an ABL inhibitor, an AXL inhibitor, a proteasome inhibitor, an anti-CCR4 antibody, a JAK inhibitor, a CXCR4 antagonist, a BET inhibitor, a Bcl-2 inhibitor, an HDAC inhibitor, a FLT3 inhibitor, an LSD1 inhibitor, an MDM2 inhibitor, an IDH1 inhibitor, an IDH2 inhibitor, a Btk inhibitor, a PI3K inhibitor, a PKC inhibitor, a SYK inhibitor, an mTOR inhibitor, an Akt inhibitor, a MEK inhibitor, an EZH2 inhibitor, a PLK inhibitor, an HSP90 inhibitor, an XPO1 inhibitor, a SMO inhibitor, a NEDD8 inhibitor, a Btk degradation inducer, an IRAK4 degradation inducer, an IRAK4 inhibitor, a CDK4/6 inhibitor, and a retinoid.
[8] The therapeutic agent against hematological cancer according to any one of [1] to [7], wherein the molecular targeted drug is a Btk inhibitor, and the Btk inhibitor is one or more selected from the group consisting of Ibrutinib, Tirabrutinib, Spebrutinib, Acalabrutinib, Evobrutinib, Poseltinib, Fenebrutinib, Vecabrutinib, Zanubrutinib, PRN-1008, BMS-986142, LOXO-305, ARQ-531, and salts thereof.
[9] The therapeutic agent against hematological cancer according to any one of [1] to [8], wherein the Btk inhibitor is Ibrutinib or a salt thereof.
[10] The therapeutic agent against hematological cancer according to any one of [1] to [8], wherein the Btk inhibitor is one or more selected from the group consisting of Tirabrutinib, Acalabrutinib, Zanubrutinib, LOXO-305, ARQ-531, and salts thereof.
[11] The therapeutic agent against hematological cancer according to any one of [1] to [10], wherein the hematological cancer is multiple myeloma, leukemia, malignant lymphoma, T-cell lymphoma, Hodgkin disease, or cancer of unknown primary.
[12] A medicine (a therapeutic agent against hematological cancer) comprising a combination of the compound represented by the formula (I) or a salt thereof, or a co-crystal, a hydrate or a solvate thereof according to [1] and a cytotoxic anticancer agent and/or a molecular targeted drug.
[13] A medicine (a therapeutic agent against hematological cancer) comprising a combination of the compound represented by the formula (I) or a salt thereof, or a co-crystal, a hydrate or a solvate thereof according to [1] and a cytotoxic anticancer agent and/or a molecular targeted drug, which are administered separately or simultaneously.
[14] A method for treating cancer, the method comprising administering to a patient in need of treating hematological cancer an effective amount of the compound represented by the formula (I) or a salt thereof, or a co-crystal, a hydrate or a solvate thereof according to [1] and an effective amount of a cytotoxic anticancer agent and/or a molecular targeted drug, separately or simultaneously.
[15] A method for treating hematological cancer, the method comprising administering an effective amount of the compound represented by the formula (I) or a salt thereof, or a co-crystal, a hydrate or a solvate thereof according to [1] to a patient in need of treating hematological cancer, the method further comprising administering an effective amount of a cytotoxic anticancer agent and/or a molecular targeted drug.
[16] A method for treating hematological cancer, the method comprising administering an effective amount of a cytotoxic anticancer agent and/or a molecular targeted drug to a patient in need of treating hematological cancer, the method further comprising administering an effective amount of the compound represented by the formula (I) or a salt thereof, or a co-crystal, a hydrate or a solvate thereof according to [1].
[17] A compound represented by the formula (I) or a salt thereof, or a co-crystal, a hydrate or a solvate thereof according to [1], which is used for treating hematological cancer in combination with a cytotoxic anticancer agent and/or a molecular targeted drug.
[18] A cytotoxic anticancer agent and/or a molecular targeted drug, which is used for treating hematological cancer in combination with the compound represented by formula (I) or a salt thereof, or a co-crystal, a hydrate or a solvate thereof according to [1].
[19] Use of a compound represented by formula (I) or a salt thereof, or a co-crystal, a hydrate or a solvate thereof according to [1] for the manufacture of a therapeutic agent against hematological cancer adopted to be administered in combination with a cytotoxic anticancer agent and/or a molecular targeted drug.
[20] Use of a cytotoxic anticancer agent and/or a molecular targeted drug for the manufacture of a therapeutic agent against hematological cancer adopted to be administered in combination with the compound represented by the formula (I) or a salt thereof, or a co-crystal, a hydrate or a solvate thereof according to [1].

All documents cited herein are incorporated herein by reference.

All of the above description is non-limiting, and the present invention is defined in the appended claims and various modifications can be made without departing from the technical idea thereof. Hereinafter, the present invention will be described in more detail with reference to Examples, but the present invention is not limited these Examples.

### EXAMPLES

Hereinafter, the present invention will be specifically described with reference to Examples, but the present invention is not limited thereto.

Biological Example 1: Evaluation of combined effect on cell viability by combination of Compound A and Btk inhibitor on human B-cell lymphoma cell line (in vitro)

### [Operation]

### (1) Cell culture

Using Ibrutinib as a Btk inhibitor, the combined effect on cell viability with Compound A was evaluated in vitro. In this evaluation test, two types of human CD79 mutation-positive diffuse large B-cell lymphoma cell lines were cultured using Iscove's modified Dulbecco's medium containing 20% inactivated calf serum and 0.5 mmol/L Monothioglycerol under conditions of 5% CO₂ and 37°C.

### (2) Evaluation of cell viability

After seeding two types of human CD79 mutation-positive diffuse large B-cell lymphoma cell lines (1250 cells/90 µL or 1000 cells/90 µL) in each well of a 96 well plate, 10 µL of a medium containing only DMSO, or Compound A (0.3 nM, 1 nM, 3 nM, 10 nM, 30 nM, 100 nM, 300 nM, 1000 nM, and 3000 nM), ibrutinib (0.3 nM, 1 nM, 3 nM, 10 nM and 30 nM), or both drugs (combination of the above concentrations) was added so that various final concentrations were rapidly attained. After 6 days of culture from the addition, the cell viability was evaluated as follows.

Cell viability was evaluated by each treatment by measuring intracellular adenosine triphosphate (ATP) using CellTiter-Glo. That is, 100 µL of CellTiter-Glo solution per well was added to each well and incubated at room temperature for 15 minutes, and then chemiluminescence was measured. The average value of each combination was calculated from the obtained data, and a concentration reaction curve of the cell viability of each drug alone or in combination was created with the luminescence intensity in cells treated with DMSO alone as 100%.

### (3) Data analysis

The half maximal inhibitory concentration (IC₅₀) for each drug was determined using median effect analysis software CalcuSyn 2.0 (CalcuSyn, Inc.). Subsequently, the combination index (CI) at the drug combination concentration was determined (according to the method described in Advances in enzyme regulation, Vol. 22, 1984, pages 27-55). The CI value is an index generally used to determine the strength of the combined effect, and has the meaning described in Table 2.

### [Results]

The evaluation results of antitumor effect by the combined treatment of Compound A and ibrutinib in each cell line are shown in Table 1. When treated with Compound A and ibrutinib in combination, the median of CI was less than 1, and a synergistic effect was exhibited.

**[Table 1]**

| Name of cell line | Ibrutinib |
|---|---|
| Human CD79 mutation-positive diffuse large B-cell lymphoma cell line 1 | 0.629 |
| Human CD79 mutation-positive diffuse large B-cell lymphoma cell line 2 | 0.504 |

**[Table 2]**

| CI | Effect |
|---|---|
| < 0.1 | Very strong synergistic effect |
| 0.1-0.3 | Strong synergistic effect |
| 0.3-0.7 | Synergistic effect |
| 0.7-0.85 | Weak synergistic effect |
| 0.85-0.90 | Very weak synergistic effect |
| 0.90-1.10 | Additive effect |
| 1.10-1.20 | Very weak antagonism |
| 1.20-1.45 | Weak antagonism |
| 1.45-3.3 | Antagonism |
| 3.3-10 | Strong antagonism |
| > 10 | Very strong antagonism |

### Biological Example 2: Evaluation of combined effect of antitumor effect by combination of Compound A and Btk inhibitor on human B-cell lymphoma cell line (in vivo)

### [Operation]

### (1) Cell culture

A human CD79 mutation-positive diffuse large B-cell lymphoma cell line was used. The cells were subcultured using Iscove's modified Dulbecco's medium containing 20% inactivated calf serum and 0.5 mmol/L Monothioglycerol under conditions of 5% CO₂ and 37°C.

### (2) Evaluation of antitumor effect in mouse subcutaneous transplantation model

The cells were suspended in HBSS: Matrigel = 1: 1 and implanted subcutaneously in the abdomen of 7-week-old female NOG mice at 1 × 10⁶ cells/mouse. The tumor volume was measured using a caliper, and calculated by a calculation formula of tumor volume = (major tumor diameter × minor tumor diameter × minor tumor diameter)/2. At the time when the tumor volume reached about 120 mm³, grouping was performed into 4 groups of 6 mice per group. Compound A was orally administered at a dose of 10 mg/kg twice a day for 21 days, and Ibrutinib was orally administered at a dose of 10 mg/kg once a day for 21 days. Also, the combined administration group was orally administered with 10 mg/kg of Compound A and 10 mg/kg of ibrutinib. The control group was orally administered with a 0.5% methylcellulose solution. After grouping, tumor volume measurements were performed twice a week. T/C (%) was used as an index of the antitumor effect. T/C (%) = (Amount of change in tumor volume in drug administration group)/ (Amount of change in tumor volume in control group) × 100

### (3) Data analysis

Regarding the amount of tumor volume change during the administration period of each group, the combined antitumor effect of Compound A and Ibrutinib was analyzed by a statistical method of two-way ANOVA of EZR(ver. 1.54) software.

### [Results]

The evaluation results of antitumor effect by single agent and combined administration of Compound A and Ibrutinib in the human CD79 mutation-positive diffuse large B-cell lymphoma cell line are shown in Table 3. The interaction when Compound A and Ibrutinib were administered in combination was significant (p = 0.001543), and it was confirmed that a synergistic effect of the antitumor effect was exhibited by the combination.

**[Table 3]**

| Group | Compound name | T/C (%) |
|---|---|---|
| 1 | Control group | - |
| 2 | Compound A 10 mg/kg | 49.8 |
| 3 | Ibrutinib 10 mg/kg | 63.1 |
| 4 | Compound A 10 mg/kg + Ibrutinib 10 mg/kg | -9.6 |

### Biological Example 3: Evaluation of antitumor effect by combination of Compound A and Btk inhibitor or Bcl-2 inhibitor on human B-cell lymphoma cell line (in vitro)

### [Operation]

### (1) Cell culture

Human CD79 mutation-positive and CARD 11 mutation-positive diffuse large B-cell lymphoma cell lines and human mantle cell lymphoma cell lines were used. The cells were subcultured using RPMI1640 containing 10 or 20% non-mobilized fetal bovine serum (hereinafter, the medium) under conditions of 5% CO₂ and 37°C.

### (2) Evaluation of cell viability

The cells suspended in the medium were seeded in 96 well plates at a density of 1250, 2500 or 5000 cells/100 µL per well. A vehicle or Compound A at a concentration 1000 times the final concentration and a Btk inhibitor or a Bcl-2 inhibitor were added thereto using a Tecan degital dispenser D300e (Tecan), and the mixture was cultured for 6 days under conditions of 5% CO₂ and 37°C. Treatment concentrations of Compound A were 10, 30 or 100 nmol/L, and treatment concentrations of the Btk inhibitor and the Bcl-2 inhibitor were set between 0.3 to 300 nmol/L or 0.003 to 10 nmol/L, respectively. After completion of the culture, the number of surviving cells was measured using Cell Counting Kit-8 (DOJINDO) according to the procedure of the kit.

### (3) Data analysis

The cell proliferation inhibition rate (%) in each compound-treated group was calculated as a relative value with the vehicle-treated group as 100%. Using the calculated cell proliferation inhibition rate (%), the Combination index (CI) value described in Advances in enzyme regulation, Vol. 22, 1984, pages 27-55 was calculated, and the combined effect of each compound was analyzed. The CI value is an index generally used to determine the strength of the combined effect, and has the meaning described in Table 2 above.

### [Results]

The evaluation results (CI values) of antitumor effect by the combined treatment of Compound A and a Btk inhibitor (Tirabrutinib, Acarabrutinib, Zanubrutinib, LOXO-305, ARQ-531) or a Bcl-2 inhibitor (Venetoclax) in each cell line are shown in Tables 4 and 5. The median of CI when treated with Compound A and a Btk inhibitor or a Bcl-2 inhibitor in combination were all less than 1, and an additive or synergistic effect was exhibited.

**[Table 4]**

| Name of cell line | Tirabrutinib | Acarabrutinib | Zanubrutinib | LOXO-305 | ARQ-531 |
|---|---|---|---|---|---|
| Human CD79 mutation-positive DLBCL cell line | 0.761 | 0.839 | 0.811 | 0.764 | 0.701 |
| Human MCL cell line | 0.822 | 0.956 | 0.75 | 0.646 | 0.928 |

**[Table 5]**

| Name of cell line | Venetoclax |
|---|---|
| Human CD79 mutation-positive DLBCL cell line | 0.5985 |
| Human CARD11 mutation-positive DLBCL cell line | 0.5995 |
| Human MCL cell line | 0.372 |

From the above results, it was confirmed that the combined use of Compound A and the Btk inhibitor or the Bcl-2 inhibitor exhibits a strong antitumor effect.

### Biological Example 4: Evaluation of antitumor effect by combination of Compound A and each anticancer agent on human B-cell lymphoma cell line (in vitro)

### [Operation]

### (1) Cell culture

Human CD79 mutation-positive diffuse large B-cell lymphoma cell lines and human mantle cell lymphoma cell lines were used. The cells were subcultured using RPMI1640 containing 10% non-mobilized fetal bovine serum (hereinafter, the medium) under conditions of 5% CO₂ and 37°C.

### (2) Evaluation of cell viability

The cells suspended in the medium were seeded in 96 well plates at a density of 2500 or 5000 cells/100 µL per well. A vehicle or Compound A at a concentration 1000 times the final concentration and various anticancer agents were added thereto using a Tecan degital dispenser D300e (Tecan), and the mixture was cultured for 6 days under conditions of 5% CO₂ and 37°C. The treatment concentration of Compound A was 3, 10, 30, or 100 nmol/L, and the treatment concentration of the anticancer agent was set between 0.178 to 10,000 nmol/L with reference to the antitumor effect of each anticancer agent alone. After completion of the culture, the number of surviving cells was measured using Cell Counting Kit-8 (DOJINDO) according to the procedure of the kit.

### (3) Data analysis

The cell proliferation inhibition rate (%) in each compound-treated group was calculated as a relative value with the vehicle-treated group as 100%. Using the calculated cell proliferation inhibition rate (%), the Combination index (CI) value described in Advances in enzyme regulation, Vol. 22, 1984, pages 27-55 was calculated, and the combined effect of each compound was analyzed. The CI value is an index generally used to determine the strength of the combined effect, and has the meaning described in Table 2 above.

### [Results]

The evaluation results (CI values) of antitumor effect by the combined treatment of Compound A and each anticancer agent in each cell line are shown in Table 6. When treated with Compound A and the anticancer agent in combination, the median of CI was about less than 1, and an additive or synergistic effect was exhibited with any anticancer agent.

**[Table 6]**

| Classification | Drug name | Human CD79 mutation-positive diffuse large B-cell lymphoma cell line | Human mantle cell lymphoma cell line |
|---|---|---|---|
| Alkylating agent | Cyclophosphamide | - | 0.931 |
| | Bendamustine | 0.796 | 0.793 |
| Anticancer antibiotic | Doxorubicin | 0.953 | 0.932 |
| Plant alkaloid | Irinotecan (SN-38) | 0.993 | 0.903 |
| | Vincristine | 0.961 | 0.860 |
| Steroid | Prednisolone | 0.168 | 0.854 |
| Immunomodulator | Lenalidomide | 0.736 | 0.190 |
| Anti-CD20 antibody | Anti-CD20 antibody | 0.936 | 0.950 |
| Proteasome inhibitor | Bortezomib | 0.961 | 0.989 |
| HDAC inhibitor | Tucidinostat | 0.756 | 0.668 |
| | Vorinostat | 0.930 | 0.866 |
| | Romidepsin | 0.886 | 0.819 |
| IRAK4 inhibitor | CA-4948 | 0.905 | 0.846 |
| PI3K inhibitor | BKM120 | 0.729 | - |
| | GDC-0941 | 0.873 | 0.694 |
| | Idelalisib | 0.528 | 0.457 |
| | Zandelisib | 0.494 | 0.698 |
| PKC inhibitor | Sotrastaurin | 0.896 | 0.775 |
| | Enzastaurin | 0.854 | 0.842 |
| SYK inhibitor | PRT062607 | 0.630 | 0.820 |
| mTOR inhibitor | Everolimus | 0.464 | 0.225 |
| | Sirolimus | 0.516 | 0.197 |
| AKT inhibitor | MK-2206 | 0.626 | 0.915 |
| MEK inhibitor | Trametinib | 0.218 | 0.971 |
| CDK4/6 inhibitor | Palbociclib | 0.658 | 0.443 |
| Retinoid | Bexarotene | - | 0.276 |

### INDUSTRIAL APPLICABILITY

The combination of the present invention is useful as a therapeutic agent against hematological cancer because it exhibits a strong antitumor effect.

This application is based on Japanese Patent Application No. 2022-015169 filed in Japan (filing date: February 2, 2022), the content of which is incorporated herein by reference in its entirety.

## Claims

1. A therapeutic agent against hematological cancer, wherein the agent is to be administered in combination with a cytotoxic anticancer agent and/or a molecular targeted drug, comprising a compound represented by a formula (I): 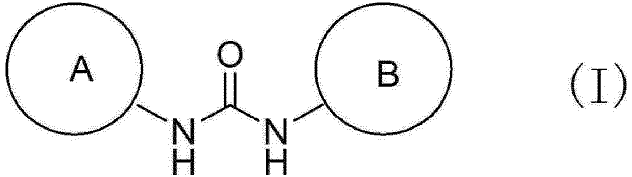
(wherein A represents 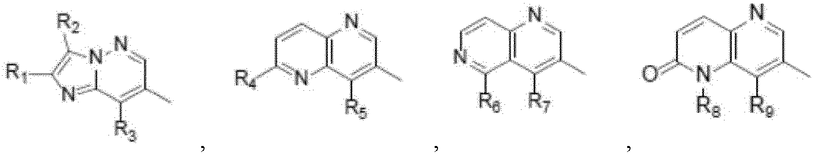 , or 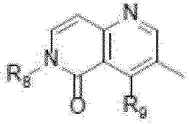 ;
R₁ represents 1) a hydrogen atom, 2) a halogen atom, 3) a cyano group, 4) a C₁₋₃ alkyl group which may be substituted with 1 to 3 halogen atoms, 5) a C₁₋₃ alkoxy group, 6) a C₃₋₆ cycloalkyl group, or 7) a phenyl group;
R₂ represents 1) a hydrogen atom or 2) a halogen atom;
R₃ represents 1) a C₁₋₆ alkyl group which may be substituted with 1 to 3 substituents selected from a C₁₋₃ alkoxy group, a hydroxyl group and a halogen atom, 2) a pyrazolyl group which may be substituted with 1 to 3 substituents selected from a C₁₋₃ alkyl group and a halogen atom, 3) a C₃₋₆ cycloalkyl group, 4) an amino group disubstituted with a C₁₋₃ alkyl group, or 5) a phenyl group which may be substituted with 1 to 3 halogen atoms;
R₄ and R₆ represent 1) a hydrogen atom, 2) a halogen atom, 3) a C₁₋₃ alkyl group which may be substituted with 1 to 3 substituents selected from a) a hydroxyl group, b) a C₁₋₃ alkoxy group which may be substituted with a 4-methoxyphenyl group and c) a halogen atom, or 4) a C₁₋₃ alkoxy group which may be substituted with 1 to 3 halogen atoms;
R₅, R₇ and R₉ represent 1) a C₁₋₆ alkyl group which may be substituted with 1 to 3 C₁₋₃ alkoxy groups or 2) a phenyl group which may be substituted with 1 to 3 halogen atoms;
R₈ represents a C₁₋₃ alkyl group; and
B represents
1) a phenyl group which may be substituted with 1 to 3 substituents selected from a) a halogen atom, b) a cyano group, c) a C₁₋₃ alkoxy group which may be substituted with 1 to 3 halogen atoms, and d) a triazolyl group,
2) a C₃₋₆ cycloalkyl group which may be substituted with 1 to 3 substituents selected from a) a C₁₋₃ alkyl group which may be substituted with 1 to 3 halogen atoms and b) a halogen atom,
3) a pyridyl group which may be substituted with 1 to 3 substituents selected from a) a halogen atom, b) a cyano group, c) a C₁₋₃ alkyl group which may be substituted with 1 to 3 halogen atoms, d) a C₁₋₃ alkoxy group which may be substituted with 1 to 3 substituents selected from a halogen atom and a C₁₋₃ alkoxy group, e) a pyrazolyl group which may be substituted with 1 to 3 C₁₋₃ alkyl groups, f) an imidazolyl group which may be substituted with 1 to 3 C₁₋₃ alkyl groups, g) a triazolyl group which may be substituted with 1 to 3 C₁₋₃ alkyl groups which may be substituted with 1 to 3 substituents selected from a C₁₋₃ alkoxy group and a halogen atom, h) an azetidinyl group, i) a pyrrolidonyl group, j) a tetrazolyl group which may be substituted with 1 to 3 C₁₋₃ alkyl groups, k) a pyrimidinyl group, and l) an oxazolyl group,
4) a pyrazolyl group which may be substituted with 1 to 3 substituents selected from a) a C₁₋₃ alkyl group which may be substituted with 1 to 3 halogen atoms, b) a C₁₋₃ alkoxy group which may be substituted with 1 to 3 halogen atoms, c) a cyano group, and d) a halogen atom, or
5) an imidazopyridyl group which may be substituted with 1 to 3 halogen atoms)
or a salt thereof, or a co-crystal, a hydrate or a solvate thereof.

2. A therapeutic agent against hematological cancer, comprising a cytotoxic anticancer agent and/or a molecular targeted drug, wherein the agent is to be administered in combination with the compound represented by the formula (I) or a salt thereof, or a co-crystal, a hydrate or a solvate thereof according to claim 1.

3. The therapeutic agent against hematological cancer according to claim 1 or 2, wherein the compound represented by the formula (I) is:
(1) (S)-N-(5-chloro-6-(difluoromethoxy)pyridin-3-yl)-N'-(8-(1-methoxyethyl)-2-methylimidazo[1,2-b]pyridazin-7-yl)urea,
(2) (S)-N-(6-chloro-4-(1-methoxyethyl)-1,5-naphthyridin-3-yl)-N'-(6-(2H-1,2,3-triazol-2-yl)-5-(trifluoromethyl)pyridin-3-yl)urea,
(3) (S)-N-(4-(1-methoxyethyl)-6-methyl-1,5-naphthyridin-3-yl)-N'-(6-(2H-1,2,3-triazol-2-yl)-5-(trifluoromethyl)pyridin-3-yl)urea,
(4) (S)-N-(5-chloro-6-(2H-1,2,3-triazol-2-yl)pyridin-3-yl)-N'-(8-(1-methoxyethyl)-2-methylimidazo[1,2-b]pyridazin-7-yl)urea,
(5) (S)-N-(5-cyano-6-(difluoromethoxy)pyridin-3-yl)-N'-(8-(1-methoxyethyl)-2-methylimidazo[1,2-b]pyridazin-7-yl)urea,
(6) (S)-N-(8-(1-methoxyethyl)-2-methylimidazo[1,2-b]pyridazin-7-yl)-N'-(6-(2H-1,2,3-triazol-2-yl)-5-(trifluoromethyl)pyridin-3-yl)urea,
(7) N-(5-chloro-6-(2H-1,2,3-triazol-2-yl)pyridin-3-yl)-N'-(8-(2-methoxypropan-2-yl)-2-methylimidazo[1,2-b]pyridazin-7-yl)urea,
(8) N-(5-chloro-6-(difluoromethoxy)pyridin-3-yl)-N'-(2-chloro-8-(propan-2-yl)imidazo[1,2-b]pyridazin-7-yl)urea, or
(9) N-(5-chloro-6-(2H-1,2,3-triazol-2-yl)pyridin-3-yl)-N'-(2-methyl-8-(propan-2-yl)imidazo[1,2-b]pyridazin-7-yl)urea.

4. The therapeutic agent against hematological cancer according to claim 3, wherein the compound represented by the formula (I) is (S)-N-(5-chloro-6-(difluoromethoxy)pyridin-3-yl)-N'-(8-(1-methoxyethyl)-2-methylimidazo[1,2-b]pyridazin-7-yl)urea.

5. The therapeutic agent against hematological cancer according to claim 3, wherein the compound represented by the formula (I) is (S)-N-(6-chloro-4-(1-methoxyethyl)-1,5-naphthyridin-3-yl)-N'-(6-(2H-1,2,3-triazol-2-yl)-5-(trifluoromethyl)pyridin-3-yl)urea.

6. The therapeutic agent against hematological cancer according to claim 1 or 2, wherein the cytotoxic anticancer agent is one or more selected from the group consisting of Cyclophosphamide, Doxorubicin, Vincristine, Bendamustine, Irinotecan, Prednisolone, and Lenalidomide.

7. The therapeutic agent against hematological cancer according to claim 1 or 2, wherein the molecular targeted drug is one or more selected from the group consisting of an anti-CD20 antibody, an anti-CD30 antibody-drug conjugate, an anti-CD33 antibody, an anti-CD38 antibody, an anti-CD47 antibody, an anti-CD52 antibody, an anti-CD70 antibody, an anti-CD79 antibody-drug conjugate, an anti-PD-1 antibody, an anti-PD-L1 antibody, an ABL inhibitor, an AXL inhibitor, a proteasome inhibitor, an anti-CCR4 antibody, a JAK inhibitor, a CXCR4 antagonist, a BET inhibitor, a Bcl-2 inhibitor, an HDAC inhibitor, a FLT3 inhibitor, an LSD1 inhibitor, an MDM2 inhibitor, an IDH1 inhibitor, an IDH2 inhibitor, a Btk inhibitor, a PI3K inhibitor, a PKC inhibitor, a SYK inhibitor, an mTOR inhibitor, an Akt inhibitor, a MEK inhibitor, an EZH2 inhibitor, a PLK inhibitor, an HSP90 inhibitor, an XPO1 inhibitor, a SMO inhibitor, a NEDD8 inhibitor, a Btk degradation inducer, an IRAK4 degradation inducer, an IRAK4 inhibitor, a CDK4/6 inhibitor, and a retinoid.

8. The therapeutic agent against hematological cancer according to claim 7, wherein the molecular targeted drug is a Btk inhibitor, and the Btk inhibitor is one or more selected from the group consisting of Ibrutinib, Tirabrutinib, Spebrutinib, Acalabrutinib, Evobrutinib, Poseltinib, Fenebrutinib, Vecabrutinib, Zanubrutinib, PRN-1008, BMS-986142, LOXO-305, ARQ-531, and salts thereof.

9. The therapeutic agent against hematological cancer according to claim 8, wherein the Btk inhibitor is Ibrutinib or a salt thereof.

10. The therapeutic agent against hematological cancer according to claim 8, wherein the Btk inhibitor is one or more selected from the group consisting of Tirabrutinib, Acalabrutinib, Zanubrutinib, LOXO-305, ARQ-531, and salts thereof.

11. The therapeutic agent against hematological cancer according to claim 1 or 2, wherein the hematological cancer is multiple myeloma, leukemia, malignant lymphoma, T-cell lymphoma, Hodgkin disease, or cancer of unknown primary.

12. A medicine comprising a combination of the compound represented by the formula (I) or a salt thereof, or a co-crystal, a hydrate or a solvate thereof according to claim 1 and a cytotoxic anticancer agent and/or a molecular targeted drug.

13. A medicine comprising a combination of the compound represented by the formula (I) or a salt thereof, or a co-crystal, a hydrate or a solvate thereof according to claim 1 and a cytotoxic anticancer agent and/or a molecular targeted drug, which are administered separately or simultaneously.

14. A method for treating hematological cancer, the method comprising administering to a patient in need of treating hematological cancer an effective amount of the compound represented by the formula (I) or a salt thereof, or a co-crystal, a hydrate or a solvate thereof according to claim 1 and an effective amount of a cytotoxic anticancer agent and/or a molecular targeted drug, separately or simultaneously.

15. A method for treating hematological cancer, the method comprising administering an effective amount of the compound represented by the formula (I) or a salt thereof, or a co-crystal, a hydrate or a solvate thereof according to claim 1 to a patient in need of treating hematological cancer, the method further comprising administering an effective amount of a cytotoxic anticancer agent and/or a molecular targeted drug.

16. A method for treating hematological cancer, the method comprising administering an effective amount of a cytotoxic anticancer agent and/or a molecular targeted drug to a patient in need of treating hematological cancer, the method further comprising administering an effective amount of the compound represented by the formula (I) or a salt thereof, or a co-crystal, a hydrate or a solvate thereof according to claim 1.

17. A compound represented by the formula (I) or a salt thereof, or a co-crystal, a hydrate or a solvate thereof according to claim 1, which is used for treating hematological cancer in combination with a cytotoxic anticancer agent and/or a molecular targeted drug.

18. A cytotoxic anticancer agent and/or a molecular targeted drug, which is used for treating hematological cancer in combination with the compound represented by the formula (I) or a salt thereof, or a co-crystal, a hydrate or a solvate thereof according to claim 1.

19. Use of a compound represented by the formula (I) or a salt thereof, or a co-crystal, a hydrate or a solvate thereof according to claim 1 for the manufacture of a therapeutic agent against hematological cancer adopted to be administered in combination with a cytotoxic anticancer agent and/or a molecular targeted drug.

20. Use of a cytotoxic anticancer agent and/or a molecular targeted drug for producing a therapeutic agent against hematological cancer adopted to be administered in combination with the compound represented by the formula (I) or a salt thereof, or a co-crystal, a hydrate or a solvate thereof according to claim 1.
